(19) Europäisches Patentamt
European Patent Office
Office européen des brevets

(11) Numéro de publication : **0 024 225 B2**

# (12) NOUVEAU FASCICULE DE BREVET EUROPEEN

(45) Date de publication du nouveau fascicule du brevet : **10.11.93 Bulletin 93/45**

(51) Int. Cl.$^5$ : **C07C 209/16,** C07C 211/08

(21) Numéro de dépôt : **80401109.6**

(22) Date de dépôt : **25.07.80**

## (54) Procédé de préparation de la diméthyléthylamine.

(30) Priorité : **14.08.79 FR 7920650**
**18.07.80 FR 8015880**

(43) Date de publication de la demande :
**25.02.81 Bulletin 81/08**

(45) Mention de la délivrance du brevet :
**07.04.82 Bulletin 82/14**

(45) Mention de la décision concernant l'opposition :
**10.11.93 Bulletin 93/45**

(84) Etats contractants désignés :
**BE DE FR GB LU NL**

(56) Documents cités :
**EP-A- 0 009 590**
**DE-A- 2 709 864**
**DE-B- 1 093 786**
**FR-A- 1 443 496**
**FR-A- 2 231 663**
**FR-A- 2 320 287**
**FR-A- 2 370 030**

(73) Titulaire : **P C U K PRODUITS CHIMIQUES UGINE KUHLMANN**
**Service Propriété Industrielle Tour Manhattan**
**F-92087 Paris La Défense 2 Cédex 21 (FR)**

(72) Inventeur : **Cheminal, Bernard**
**Le Clos Grillet**
**F-73130 La Chambre (FR)**
Inventeur : **Kiener, Paul**
**15 Chemin de la Citadelle**
**F-69230 Saint Genis Laval (FR)**
Inventeur : **Traversaz, Aimé**
**Les Brouves**
**F-73130 La Chambre (FR)**

(74) Mandataire : **Leboulenger, Jean**
**ATOCHEM Département Propriété Industrielle**
**F-92091 Paris la Défense 10 Cédex 42 (FR)**

EP 0 024 225 B2

## Description

Procédé de préparation de la diméthyléthylamine

La présente invention concerne un procédé de préparation de la diméthyléthylamine :

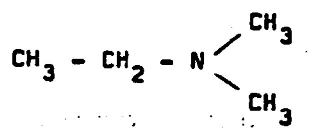

par réaction de l'éthanol avec la diméthylamine selon la réaction :

$$C_2H_5OH + (CH_3)_2NH \rightarrow C_2H_5\text{-}N(CH_3)_2 + H_2O$$

Cette réaction est effectuée en présence d'hydrogène et d'un catalyseur d'hydrogénation-déshydrogénation.

La diméthyléthylamine est un produit industriel utilisé comme catalyseur de polymérisation de résines polyuréthannes utilisées principalement dans la fabrication de moules de fonderie selon le procédé dit « boîte froide ».

L'animation catalytique des alcools, en présence de catalyseurs d'hydrogénation-déshydrogénation, et, le cas échéant, d'hydrogène est déjà connue en particulier par Houben-Weyl, vol. 11/1 pages 126 et suivantes.

De manière plus précise, A. Baiker et W. Richarz (Industrial and Engineering Chemistry, Product Research and Developpement, 1977, 16(3), pages 261 à 266) décrivent l'amination catalytique des alcools aliphatiques à longue chaîne en présence de divers catalyseurs dont les catalyseurs à base de cuivre et d'oxyde de chrome.

Cependant, lors de la diméthylamination du n-dodécanol par exemple, citée dans le tableau III de la page 265 de cet article, ces auteurs utilisent un rapport molaire diméthylamine/dodécanol = 5,5, ce qui entraîne lors d'une exploitation industrielle de ce procédé un recyclage très important de la diméthylamine en excès, d'où une perte de productivité et une consommation d'énergie accrue sous forme de vapeur. La tenue du catalyseur dans le temps n'est en outre pas mentionnée.

Le brevet français 2 320 287 décrit la réaction, en phase liquide, d'alcools à longue chaîne avec des amines primaires ou secondaires N-méthylées en présence de chromite de cuivre. Cependant, les temps de réaction sont longs (5 à 7 heures dans les exemples d'opérations discontinues), et l'alkylamination est réalisée dans un appareillage complexe, ce qui entraîne une faible productivité et un entretien coûteux. L'utilisation de catalyseurs en poudre très fine implique des temps de filtration extrêmement longs, surtout en présence de produits visqueux comme les amines N-méthylées à longue chaîne. Les exmples 1 à 8, donnant une fourchette de concentration de diméthylamine dans le gaz de circulation constitué d'hydrogène pour l'essentiel, ne permettent pas de calculer avec précision le rapport molaire diméthylamine/alcool (donc la consommation réelle en diméthylamine), bien que l'on puisse estimer que celui-ci se situe aux alentours de 1, c'est-à-dire de la théorie. Aucune indication n'est donnée par ailleurs sur la durée de vie des catalyseurs employés. Enfin la température de la réaction est telle que cette technique ne s'adapte pas aux alcools à bas point d'ébullition tels que l'éthanol, d'une part parce que son utilisation exigerait une pression de travail élevée pour travailler en phase liquide et d'autre part parce que les premiers termes des alcools aliphatiques se déshydrogènent à beaucoup plus haute température que les alcools à chaînes plus longues.

Aussi le but de la présente invention est de développer un procédé de préparation économique de la diméthyléthylamine par diméthylamination de l'éthanol en phase gazeuse, procédé qui fournit des rendements éleves de cette amine, avec des conversions pratiquement complètes.

L'invention consiste à faire réagir, en continu, l'éthanol à l'état gazeux, en présence de catalyseurs constitués de cuivre, d'oxyde de cuivre et d'oxyde de chrome, et éventuellement d'oxydes de baryum, de zinc et/ou de magnésium, sous une pression comprise entre 1 et 15 bars absolus, de préférence voisins de 8 bars absolus, à une température comprise entre 180 et 240°C, avec la diméthylamine, en mélange gazeux avec des quantités d'hydrogène telles que le rapport molaire hydrogène/éthanol soit compris entre 3 et 10, et de préférence voisin de 5, alors que le rapport molaire diméthylamine/éthanol est compris entre 0,20 et 0,60 et de préférence entre 0,25 et 0,40.

Il est possible d'obtenir ainsi la diméthyléthylamine avec un taux de conversion quantitatif de la diméthylamine et de l'éthanol théoriquement transformable, avec un rendement de 97 % par rapport à la diméthylamine et 97 % par rapport à l'éthanol-transformé, les produits secondaires présents dans le mélange réactionnel ne perturbant pas l'isolement ultérieur d'une diméthyléthylamine ayant un degré de pureté élevé. Par ailleurs la quantité de ces produits secondaires, obtenus par déshydrogénation de l'éthanol en excès et autoconden-

sation de l'acétaldéhyde ainsi généré ou par réaction de ce même acétaldéhyde sur les composés de dismutation de la diméthylamine est faible et leur pourcentage pondéral par rapport à la diméthyléthylamine fabriquée peut être maintenu à une valeur inférieure à 3 %. L'acétaldéhyde peut d'ailleurs être ultérieurement soit détruit par l'hydrate d'hydrazine soit réhydrogéné catalytiquement en éthanol à plus basse température (100 à 130°C) sous la même pression (8 bars absolus) ou une pression voisine. Les produits secondaires aminés peuvent être aisément séparés de la diméthylamine par distillation.

Le procédé de l'invention peut être mis en oeuvre de la manière décrite ci-après. La diméthylamine (stockée à l'état liquide dans un réservoir maintenu sous pression d'azote) et l'éthanol liquide sont introduits de façon continue dans un évaporateur constitué par exemple d'un tube chemisé chauffé à la vapeur et sont mélangés à l'hydrogène dans les proportions désirées et ce de la façon la plus homogène possible. Le mélange gazeux traverse ensuite le réacteur constitué d'un tube chauffé par un bain de sels fondus maintenu à une température comprise entre 180 et 240°C et de préférence voisine de 200°C contenant le catalyseur. Le temps de contact des gaz dans le réacteur peut être évalué à quelques secondes (2 à 10 secondes suivant les conditions opératoires). La pression à l'intérieur du réacteur et de ses annexes est maintenue entre 1 et 15 bars absolus, et de préférence voisine de 8 bars absolus.

L'effluent réactionnel gazeux traverse une série de condenseurs refroidis à l'eau, puis un séparateur où le liquide obtenu est soutiré en continu et où les gaz non condensés (constitués essentiellement d'hydrogène et de 1 à 2 % en volume de diméthyléthylamine) sont recyclés vers l'évaporateur à l'aide d'une pompe à circulation après avoir traversé une colonne d'absorption pouvant fonctionner soit en laveur (en arrosant le gaz chargé d'amine avec de l'eau ou de l'éthanol) soit en dévésiculeur pour débarrasser ce gaz de toute particule liquide. Etant donné qu'au cours du procédé de faibles quantités de produits secondaires gazeux principalement constitués d'hydrocarbures sont formées il est nécessaire de remplacer partiellement le gaz de recyclage par de l'hydrogène frais (environ 0,1 à 1 % et de préférence 0,2 à 0,7 % en volume).

Contrairement aux indications données dans le brevet français n° 2 320 287 déjà cité, qui revendique un rapport molaire diméthylamine/alcool voisin de 1 ou un peu supérieur, la limite supérieure de ce rapport pour la présente invention se situe vers 0,40 ; au-delà, des quantités très importantes de produits secondaires aminés (triméthylamine, méthyldiéthylamine, méthyléthylamine, diméthylbutylamine) se forment, entraînant une baisse intolérable des valeurs des rendements et gênant considérablement l'isolement ultérieur de la diméthyléthylamine. L'emploi d'un tel rapport molaire entraîne toutefois une légère formation d'acétaldéhyde qui à la différence des produits secondaires aminés peut être retransformé en éthanol par une post-hydrogénation catalytique à basse température (100 à 130 °C). Un autre moyen permettant d'éliminer les très faibles quantités de ce sous-produit consiste à traiter le mélange réactionnel par de l'hydrate d'hydrazine en ajoutant au maximum 3 fois la quantité stoechiométrique nécessaire pour transformer l'acétaldéhyde.

Les catalyseurs utilisés sont des catalyseurs au cuivre, oxyde de cuivre et oxyde de chrome, additionnés ou non d'oxyde de baryum et d'autres oxydes et supportés de manière telle que la teneur en matière active soit comprise entre 85 et 95 % c'est-à-dire avec 5 à 15 % de support. Convient en particulier un catalyseur dont la composition pondérale, après réduction, est la suivante :

- Cuivre : 31 %
- Chrome : 25 %
- Baryum : 10 %

c'est-à-dire un rapport atomique (Cu/Cr) = 1,02 et un rapport atomique (Ba/Cu) = 0,15.

Les catalyseurs sont supportés de préférence par un support inerte comme la silice, et mis sous forme de grains, extrudats, billes ou comprimés, mais de préférence sous forme de pastilles cylindriques de 3 à 6 mm de diamètre et d'épaisseur. L'espérance de vie d'une charge catalytique peut être estimée à environ 2000 heures (3 mois).

Le catalyseur peut être régénéré par une oxydation des matières carbonées par un mélange d'air dilué dans de l'azote suivie d'une réduction par un mélange d'hydrogène et de vapeur d'eau. Le catalyseur régénéré présente une très légère baisse d'activité par rapport à son activité à l'état neuf, mais cette méthode permet d'effectuer des campagnes de fabrication de l'ordre de plusieurs mois.

Lorsque la post-catalyse est mise en oeuvre, il est possible d'utiliser des catalyseurs identiques à ceux de la réaction principale ou des catalyseurs d'hydrogénation différents, tels que le nickel Raney foraminé en morceaux de 2 à 10 mm, éventuellement déposé sur un support de silice, utilisé en pastilles de 3 à 10 mm.

Les exemples suivants illustrent la présente invention sans toutefois la limiter.

Exemple 1

La réaction est effectuée dans l'appareillage illustré par la figure. L'appareil consiste en un évaporateur (1) où sont introduits la diméthylamine anhydre liquide par la conduite (2) l'éthanol azéotropique liquide par la

conduite (3) et le gaz de recyclage par la conduite (4), un réacteur monotubulaire (5) de 2 litres, deux condenseurs en série refroidis à l'eau (6), un séparateur (7), un laveur (ou dévésiculeur) (8), dont la phase liquide est reliée au séparateur par la conduite (9), une conduite (10) d'évacuation des gaz, une conduite (11) d'amenée d'hydrogène frais sous pression, une pompe (12) de recyclage des gaz et une conduite (15) d'alimentation en azote. Le chauffage du réacteur est assuré par un bain de sels fondus contenu dans la cuve (13). Une conduite (16) permet d'amener de l'eau pour la réduction du catalyseur. L'évaporateur est chauffé à la vapeur à l'aide de la chemise (14). Le réacteur est équipé d'une gaine thermométrique (17) qui permet de mesurer le profil de température tout le long du tube réactionnel et à l'intérieur du lit catalytique au moyen d'un thermocouple. Cet équipement sert d'une part à suivre l'évolution de la température lors de la réduction du catalyseur et d'autre part à examiner en cours de marche l'évolution du degré d'avancement de la réaction tout au long du réacteur monotubulaire.

Dans le réacteur (5) de 2 litres on place 2 800 g de catalyseur au chromite de cuivre (31 % de Cu, 25 % de Cr, 10 % de Ba) en pastilles de 4,5 x 4,5 mm, on balaie l'appareil avec de l'azote par la conduite (15) puis par les conduites (11) et (16) avec de l'hydrogène et de la vapeur d'eau dans un rapport molaire de l'ordre de 20 à 25, tel que la pointe de température à l'intérieur du lit catalytique ne dépasse pas 250 à 260 °C pour une température du bain de sels contenu dans la cuve (13) de 230°C. Lorsque la pointe de température arrive au sommet du lit du catalyseur la réduction de celui-ci est terminée et la température du bain est amenée à 200 °C environ. La pression absolue de l'installation est amenée à 4 bars au moyen d'hydrogène par la conduite (11) et la pompe (12) mise en marche de façon à réaliser dans l'installation une circulation de gaz à un débit de 2 780 Nl/h (124 moles $H_2$/h) cette pression étant maintenue constante. On injecte alors par la conduite (2) la diméthylamine liquide anhydre à un débit de 424 g/h (9,4 moles/h) et l'éthanol azéotropique par la conduite (3) à un débit (calculé en éthanol pur) de 1 428 g/h (31 moles/h). Cet essai est donc réalisé avec les rapports molaires suivants :

Diméthylamine/éthanol = 0,30
Hydrogène/éthanol = 4

Une purge du gaz de recyclage par la conduite (10) à un débit de 18 Nl/h permet d'évacuer régulièrement les sous-produits gazeux et de maintenir une pression constante dans l'appareil en remplaçant les gaz éliminés par de l'hydrogène frais au moyen de la conduite (11). Le profil de température mesuré à l'aide du thermocouple coulissant dans la gaine (17) fait apparaître une température maximale de 205,5 °C à 50 cm du bas du lit catalytique (dont la hauteur totale est de 3 m). L'effluent réactionnel liquide est soutiré en continu par la conduite (18) pendant 1 heure. On obtient ainsi un mélange brut contenant 670 g de diméthyléthylamine et 990 g d'éthanol non transformé. Par rapport à la quantité théoriquement transformable en diméthyléthylamine, l'alcool a réagi avec un taux de 102,3 %. La diméthylamine a été totalement transformée et le rendement en diméthyléthylamine est de 97,3 %, tandis que le rendement par rapport à l'éthanol transformé est de 96,2 %. Les pourcentages pondéraux en sous-produits, exprimés par rapport à la diméthyiéthylamine formée, sont les suivants :

- triméthylamine : 1 %
- méthyldiéthylamine : 1,4 %
- diméthylbutylamine : 0,6 %

L'acétaldéhyde généré correspond à 0,4 % de la diméthyléthylamine produite. Cette teneur gênante pour la séparation par distillation de la diméthyléthylamine pure peut être diminuée de façon notable en traitant l'effluent gazeux du réacteur (5) vers 100 °C sur le même catalyseur et sous une pression absolue de 4 bars. Il est également possible de se débarrasser de cette impureté par un traitement approprié à l'hydrate d'hydrazine.

Exemple 2

Cet essai est effectué dans le même appareillage et avec le même catalyseur que ceux spécifiés dans l'exemple 1 dans les conditions opératoires suivantes :

température du bain : 210,5°C
débit de diméthylamine : 466 g/h (10,33 moles/h)
débit d'éthanol pur : 1 380 g/h (29,95 moles/h)
débit d'hydrogène : 3 360 Nl/h (150 moles/h)
c'est-à-dire avec les rapports molaires suivants :
Diméthylamine/éthanol : 0,35
Hydrogène/éthanol: 5

En opérant comme dans l'exemple 1, les résultats obtenus sont les suivants:

taux de conversion de l'éthanol théoriquement transformable = 101 %
taux de conversion de la diméthylamine = 100 %

rendement en diméthyléthylamine par rapport à la dimethylamine engagée = 97,0 %
rendement en diméthyléthylamine par rapport à l'éthanol transformé = 93,3 %
pourcentages pondéraux des principaux sous-produits par rapport à la diméthyléthylamine formée dans l'effluent réactionnel brut :

- triméthylamine = 1,1 %
- méthyldiéthylamine = 1,6 %
- diméthylbutylamine = 0,6 %

Ces résultats ont été obtenus après 185 heures de marche continue sur la même charge de catalyseur.

Exemple 3

Dans un réacteur monotubulaire analogue à celui décrit dans l'exemple 1 mais d'une capacité de 7 litres, on place 9 800 g du catalyseur décrit dans l'exemple 1, en pastilles de 4,5 x 4,5 mm. L'essai est réalisé dans les conditions opératoires suivantes :

température du bain de sels = 200 °C
pression absolue = 4 bars
débit d'éthanol (en éthanol pur) = 4 631 g/h (100,52 moles/h)
débit de diméthylamine = 1 519 g/h (33,68 moles/h)
débit d'hydrogène = 11 200 Nl/h (500,0 moles/h)

ce qui correspond aux rapports suivants :

rapport molaire diméthylamine/éthanol = 0,34
rapport molaire hydrogène/éthanol = 5

Les résultats suivants sont obtenus :

taux de conversion de l'éthanol théoriquement transformable = 102,7 %
rendement en diméthyléthylamine par rapport à l'éthanol transformé = 95,2 %
taux de conversion de la diméthylamine = 100 %
rendement en diméthyléthylamine par rapport à la diméthylamine engagée = 96,9 %

Les pourcentages pondéraux rapportés à la diméthyléthylamine fabriquée sont les suivants :

- triméthylamine = 0,8 %
- méthyldiéthylamine = 1,1 %
- acétaldéhyde = 0,7 %

Exemple 4

Cet exemple illustre l'obtention d'un effluent pratiquement débarrassé d'acétaldéhyde par traitement post-catalytique de l'effluent issu du réacteur (5).

La réaction principale réalisée sur la même charge de catalyseur que celle décrite dans l'exemple 2 et dans les mêmes conditions, mais avec un rapport molaire :

diméthylamine/éthanol = 0,33

donne les résultats suivants, obtenus après analyse d'un échantillon instantané :

pourcentages pondéraux en sous-produits par rapport à la diméthyléthylamine obtenue :

- triméthylamine = 1 %
- méthyldiéthylamine = 1,5 %
- diméthylbutylamine = 0,2 %
- acétaldéhyde = 2,2 %

L'effluent réactionnel liquide obtenu dans le réacteur (5) à l'issue de l'essai décrit ci-dessus est retraité dans un appareil analogue à celui de l'exemple 1 mais ne comportant pas de recyclage de gaz et dont le réacteur monotubulaire chauffé par une résistance électrique et non par un bain de sels fondus à une capacité de 0,5 litre. L'évaporateur est également chauffé par une résistance électrique. Dans ce réacteur on place 700 g du même catalyseur chromite de cuivre que celui décrit dans l'exemple 1 et on le réduit de manière analogue, mais en remplaçant la vapeur d'eau par de l'azote. On alimente l'effluent liquide ci-dessus à un débit de 660 g/h avec un débit d'hydrogène de 750 Nl/h (33,5 moles $H_2$/h). Le réacteur est maintenu sous une pression absolue de 4 bars et à une température de 100 °C. Le produit brut ainsi traité est recueilli à l'état liquide et l'analyse d'un échantillon instantané est pratiquée.

La teneur en acétaldéhyde par rapport à la diméthylethylamine est inférieure à 0,1 % en poids et la légère coloration jaune de l'effluent du réacteur (5) obtenue dans la première phase de l'opération a disparu, le produit obtenu après ce traitement de post-catalyse étant parfaitement incolore.

Le rendement de la diméthyléthylamine par rapport à l'éthanol transformé passe de 93,7 % dans l'étape

principale à 96,4 % après post-catalyse, ce qui prouve que l'acétaldéhyde résiduel a pu être facilement réhydrogéné en éthanol lors de la seconde étape.

Exemples 5 à 12

Afin de montrer l'influence du rapport molaire diméthylamine/éthanol sur le rendement en produit désiré donc sur l'économie du procédé, on effectue les essais suivants :

On place 0,5 litre de catalyseur chromite de cuivre en pastilles dans le réacteur décrit dans la seconde phase de l'exemple 4, on le réduit de la manière habituelle et on fait passer dessus des mélanges variés d'hydrogène, éthanol et diméthylamine gazeux sous une pression absolue de 4 bars. On effectue ainsi 4 séries d'essais sur des catalyseurs chromites de cuivre analogues.

Les deux essais d'une première série ont été réalisés sur un catalyseur chromite de cuivre dont la composition atomique est la suivante :

rapport atomique Cu/Cr = 0,8

rapport atomique Ba/Cu = 0,10

utilisé sous forme de pastilles de 3,2 × 3,2 mm.

A la température de 180 °C, avec un débit d'éthanol pur de 111 g/h (2,4 moles/h) et un rapport molaire hydrogène/éthanol = 3, on obtient les résultats suivants:

Tableau I

| Exemple | Rapport molaire diméthylamine / éthanol | Taux de conversion de l'éthanol | Rendement en diméthyléthylamine par rapport a l'éthanol | Taux de conversion de la diméthylamine (théoriquement transformable) | Rendement en diméthyléthylamine par rapport à la diméthylamine % | Rendement en sous-produit par rapport à la diméthylamine % | | |
|---|---|---|---|---|---|---|---|---|
| | | | | | | triméthylamine | méthyléthylamine | méthyldiéthylamine |
| 5 | 3,0 | 52,6 | 95,0 | 59,0 | 74,0 | 9,0 | 3,6 | 0,8 |
| 6 | 1,5 | 63,7 | 89,2 | 71,8 | 76,6 | 8,0 | 2,7 | 2,1 |

Les exemples 7 et 8 ont été effectués sur un catalyseur chromite de cuivre dont la composition atomique est la suivante :

rapport atomique Cu/Cr = 0,009

rapport atomique Ba/Cu = 0,01

utilisé sous forme de pastilles de 5 mm × 5 mm.

A la température de 200 °C, avec un débit d'éthanol pur de 228 g (5,0 moles/h) et un rapport molaire hydrogène/éthanol = 3 on obtient les résultats suivants :

Tableau II

| Exemple | Rapport molaire diméthylamine / éthanol | Taux de conversion de l'éthanol | Rendement en diméthyléthylamine par rapport à l'éthanol | Taux de conversion de la diméthylamine (théoriquement transformable) | Rendement en diméthyléthylamine par rapport à la diméthylamine % | Rendement en sous-produit par rapport à la diméthylamine % | | |
|---|---|---|---|---|---|---|---|---|
| | | | | | | triméthylamine | méthyléthylamine | méthyldiéthylamine |
| 7 | 1,5 | 59,5 | 79,6 | 46,8 | 75,0 | 7,8 | 3,7 | 2,1 |
| 8 | 1,1 | 60,3 | 74,9 | 61,5 | 73,4 | 6,2 | 2,7 | 3,5 |

Les exemples 9 et 10 ont été effectués sur le même catalyseur que celui décrit dans les exemples 7 et 8.

A la température de 215 °C, avec un débit d'éthanol pur de 5 226 g/h (4,9 moles/h), et un rapport molaire hydrogène/éthanol = 7,3 on obtient les résultats suivants :

Tableau III

| Exemple | Rapport molaire diméthylamine / éthanol | Taux de conversion de l'éthanol | Rendement en diméthyléthylamine par rapport à l'éthanol | Taux de conversion de la diméthylamine (théoriquement transformable) | Rendement en diméthyléthylamine par rapport à la diméthylamine % | Rendement en sous-produit par rapport à la diméthylamine % | | |
|---|---|---|---|---|---|---|---|---|
| | | | | | | triméthylamine | méthyléthylamine | méthyldiéthylamine |
| 9 | 0,5 | 103,9 | 85,4 | 99,2 | 88,9 | 1,5 | 0,1 | 4,8 |
| 10 | 0,3 | 96,9 | 87,4 | 100,0 | 85,3 | 0,9 | 0 | 4,1 |

Les exemples 11 et 12 ont été réalisés sur un catalyseur chromite de cuivre dont la composition atomique est la suivante :

rapport atomique Cu/Cr = 1,5

rapport atomique Ba/Cu = 0

A la température de 180 °C, avec un débit d'éthanol pur de 341 g/h (7,4 moles/h), et un rapport molaire hydrogène/éthanol = 6,5 on obtient les résultats suivants :

Tableau IV

| Exemple | Rapport molaire diméthylamine / éthanol | Taux de conversion de l'éthanol | Rendement en diméthyléthylamine par rapport à l'éthanol | Taux de conversion de la diméthylamine (théoriquement transformable) | Rendement en diméthyléthylamine par rapport à la diméthylamine % | Rendement en sous-produit par rapport à la diméthylamine % | | |
|---|---|---|---|---|---|---|---|---|
| | | | | | | triméthylamine | méthyléthylamine | méthyldiéthylamine |
| 11 | 0,5 | 91,0 | 87,6 | 88,7 | 89,8 | 2,5 | 0,8 | 4,2 |
| 12 | 0,3 | 103,5 | 87,6 | 100,0 | 94,4 | 1,2 | 0 | 2,3 |

On peut voir d'après les tableaux I à IV que, quels que soient la composition atomique du catalyseur chromite de cuivre utilisé, la température, le débit d'éthanol et le rapport molaire hydrogène/éthanol, l'influence du rapport molaire diméthylamine/éthanol est prépondérante. Ce fait est particulièrement net dans les exemples 11 et 12, où l'on voit que la valeur optimale du rapport molaire diméthylamine/éthanol se situe vers 0,30. Il faut ajouter que la présence de méthyléthylamine dans l'effluent brut nuit directement à la qualité de la diméthyléthylamine pure commerciale, car cette impureté, dont le point d'ébullition est très proche de celui de la diméthyléthylamine ne peut être séparée de cette dernière par distillation. Le fait de travailler avec un excès molaire d'éthanol par rapport à la diméthylamine entraîne la formation d'acétaldéhyde dont nous avons vu dans l'exemple 4 qu'il pouvait être entièrement réhydrogéné en éthanol, donc récupéré, ce qui a entre autres conséquences une augmentation du rendement de la diméthyléthylamine par rapport à l'éthanol.

Exemple 13

Cet exemple illustre l'influence de la pression sur les résultats.

On effectue le même essai que celui décrit dans l'exemple 2, mais avec les conditions opératoires suivantes :

température du bain : 202 °C

pression absolue : 8 bars

débit de diméthylamine : 637 g/h (14,12 moles/h)

débit d'éthanol pur : 2 123 g/h (46,09 moles/h)

débit d'hydrogène : 5 150 Nl/h (230 moles/h)

c'est-à-dire avec les rapports molaires suivants :

diméthylamine/éthanol : 0,31

hydrogène/éthanol : 5

Voici les résultats obtenus :

taux de conversion de l'éthanol théoriquement transformable = 101,8 %

rendement en diméthyléthylamine par rapport à l'éthanol transformé = 97,8 %

taux de conversion de la diméthylamine = 100,0 %

rendement en diméthyléthylamine par rapport à la diméthylamine engagée = 98,0 %

Les pourcentages pondéraux rapportés à la diméthyléthylamine fabriquée sont les suivants :

- triméthylamine : 0,9 %
- méthyldiéthylamine : 0,9 %
- triéthylamine : 0,07 %
- acétaldéhyde : 0,4 %
- méthanol : 0,1 %

La productivité correspondante est augmentée d'environ 50 % par rapport à une marche sous 4 bars absolus dans les mêmes conditions opératoires.

Exemple 14

Dans un réacteur monotubulaire analogue à celui décrit dans l'exemple 1, mais d'une capacité de 7 litres, on place 11 060 g d'un catalyseur chromite de cuivre (43 % de CuO, 43 % de $Cr_2O_3$, 8 % de BaO) en pastilles de 4,8 × 4,8 mm. Après avoir opéré la réduction du catalyseur, comme décrit dans l'exemple 1, on effectue l'essai dans les conditions suivantes :

température du bain : 210 °C

pression absolue : 8 bars

débit de diméthylamine : 2 132 g/h (47,28 moles/h)

débit d'éthanol (en produit pur) : 7 284 g/h (158,12 moles/h)

débit d'hydrogène : 15 859 Nl/h (708 moles/h)

c'est-à-dire avec les rapports molaires suivants :

diméthylamine/éthanol = 0,30

hydrogène/éthanol = 4,5

Les résultats obtenus sont les suivants :

taux de conversion de l'éthanol théoriquement transformable = 99,4 %

taux de conversion de la diméthylamine = 100 %

rendement en diméthyléthylamine par rapport à la diméthylamine engagée = 97,0 %

rendement en diméthyléthylamine par rapport à l'éthanol transformé = 97,0 %

Exemple 15

Cet exemple illustre l'obtention d'un effluent incolore débarrassé d'acétaldéhyde par traitement post-catalytique de l'effluent liquide, débarrassé des incondensables, issu du réacteur de catalyse et contenant des proportions variables d'acétaldéhyde.

Ce traitement est effectué par hydrogénation de l'acétaldéhyde en phase liquide selon une technique dite « en phase ruisselante » où le liquide à traiter et l'hydrogène sous pression, parcourent de haut en bas, à co-courant, un lit de catalyseur d'hydrogénation.

L'efficacité du traitement est suivie au moyen d'un test qualitatif dit du « miroir d'argent » qui permet de déceler des traces d'aldéhydes.

Sur 0,5 litre de catalyseur nickel Raney foraminé en grains de 2 à 5,5 mm, disposé dans un réacteur tubulaire dont la double enveloppe est parcourue par de la vapeur, d'un diamètre intérieur de 25 mm, on alimente au moyen d'une pompe l'effluent liquide du réacteur de catalyse obtenu au cours de l'essai décrit dans l'exemple 14. Ce catalyseur a été obtenu par attaque sodique (au moyen d'une solution aqueuse d'hydroxyde de sodium à 50 g/l) à 100 °C, de 40 % en poids de l'aluminium contenu dans l'alliage de Raney (50 % de nickel-50 % d'aluminium) de départ.

L'effluent liquide légèrement coloré est réchauffé vers 120 °C et traité sur ce catalyseur à un débit de 10,7 l/h à 116 °C sous une pression de 8 bars absolus, en présence d'hydrogène dont le débit de purge est d'environ 50 Nl/h. Après 6 heures de marche continue dans ces conditions opératoires, aucune apparition d'acétaldéhyde ne s'est manifestée, et l'effluent post-catalytique obtenu est parfaitement incolore, ce qui permet d'obtenir par distillation un produit commercial satisfaisant.

Il est possible également d'opérer de la manière suivante: sur 0,5 litre d'un catalyseur à base de nickel (45 %) déposé sur silice, en pastilles de 5 × 5 mm, préréduit dans un mélange eau-isobutanol.

On alimente avec l'effluent liquide du réacteur de catalyse décrit dans l'exemple 14, qui contient 0,02 mole d'acétaldéhyde/kg, à un débit de 12 litres/h, et on ne décèle plus aucune trace d'aldéhyde à la sortie du réacteur de post-catalyse, l'effluent ainsi obtenu étant également devenu parfaitement incolore; il peut alors être soumis à une distillation sans aucun inconvénient.

Exemple 16

Cet exemple illustre la possibilité d'un fonctionnement continu de longue durée d'un réacteur de catalyse chargé du catalyseur décrit dans l'exemple 1 couplé à un réacteur de post-catalyse tel que décrit dans l'exemple 15. Cet ensemble catalytique fournit un effluent réactionnel débarrassé d'acétaldéhyde et parfaitement incolore.

Le réacteur de catalyse contient 2 litres (~ 2 800 g) du catalyseur décrit dans l'exemple 1 (chromite de cuivre).

Le réacteur de post-catalyse qui reçoit l'effluent réactionnel liquide du premier réacteur est chargé de 0,75 litre d'un catalyseur à base de nickel (45 %) déposé sur silice en extrudats de 5 mm de longueur et 3 mm de diamètre, préréduit dans un mélange eau-isobutanol.

Les conditions de marche du catalyseur (en pastilles de 4,5 × 4,5 mm), réduit comme dans l'exemple 1, sont les suivantes :

pendant 750 heures :
température du bain de sels : 200 °C
pression absolue : 8 bars
débit d'éthanol pur : 2 073 g/h (45,0 moles/h)
débit de diméthylamine : 649 g/h (14,4 moles/h)
débit d'hydrogène : 5 342 Nl/h (239 moles/h)

c'est-à-dire avec les rapports molaires suivants :
diméthylamine/éthanol = 0,32
hydrogène/éthanol = 5,3

La température du bain a ensuite été relevée de 2 °C jusquà la 785e heure de marche, puis à nouveau de 3 °C, soit 205 °C jusqu'à la 942e heure de fonctionnement. A la 850e heure de marche le rapport molaire hydrogène/éthanol a été ramené à 4. A la 942e heure de marche, la température du bain a été redescendue à 200 °C.

Le post-catalyseur au nickel déposé sur silice a fonctionné pendant 845 heures dans les conditions suivantes :

température : 112 °C
pression absolue : 7 bars
débit de liquide condensé à traiter (contenant 0,02 mole d'acétaldéhyde/kg) = 3,6 litres/h.

Les résultats moyens, qui suivent, ont été obtenus :

taux de conversion de l'éthanol théoriquement transformable : 100,5 %
taux de conversion de la diméthylamine : 99,9 %
rendement en diméthyléthylamine par rapport à l'éthanol transformé : 97,4 %
pourcentages pondéraux des principaux sous-produits par rapport à la diméthyléthylamine formée dans l'effluent réactionnel brut (à la sortie du réacteur de post-catalyse).

- triméthylamine : 0,97 %
- méthyldiéthylamine : 0,89 %
- triéthylamine : 0,13 %
- diméthylbutylamine : 0,18 %
- méthanol : 0,10 %
- diméthylamine résiduelle : 0,07 % (contre 0,5 à 1 % à la sortie du réacteur de catalyse)
- acétaldéhyde à la sortie du réacteur de catalyse : 0,011 à 0,036 mole/kg
- acétaldéhyde à la sortie du réacteur de post-catalyse: néant (test au miroir d'argent négatif).

Aucune baisse d'activité n'a été enregistrée en 845 heures de fonctionnement du post-catalyseur. Par ailleurs, la légère perte d'activité du catalyseur en 950 heures de marche a pu être efficacement compensée par une baisse du rapport molaire hydrogène/éthanol.

Exemple 17

Cet exemple illustre la possibilité de régénérer le catalyseur principal par oxydation des matières carbonées par un mélange constitué d'air dilué dans de l'azote et réduction ultérieure par un mélange d'hydrogène

et de vapeur d'eau comme dans l'exemple 1 et dans le même appareillage.

Le mode opératoire choisi pour cette oxydation est le suivant:

température du bain de sels: 200 °C
pointe de température mesurée dans la gaine du réacteur: 300 °C
rapport molaire azote/air réglé de façon à ne pas dépasser la température de 300°C
débit d'eau (initiateur d'oxydation): 80 g pendant 45 mn.

La pointe de température se déplace alors régulièrement vers le haut du catalyseur, pendant une durée de 5 heures.

Le catalyseur est ensuite porté sous un courant d'air pur à 450°C puis la réduction menée à 230°C comme dans l'exemple 1.

Un test de l'activité du catalyseur régénéré a été effectué dans les conditions opératoires de l'exemple 16, pendant 4 heures. Une très légère baisse d'activité a été enregistrée par rapport au catalyseur neuf, non traité par oxydation (pourcentage pondéral en diméthylamine non transformée par rapport à la diméthyléthylamine obtenue: 0,2 %).

Exemple 18

Cet exemple illustre l'influence de la nature du catalyseur sur les résultats. L'essai a été effectué dans un réacteur identique à celui de l'exemple 1, en présence de 2 litres d'un catalyseur contenant un faible pourcentage d'oxyde de potassium, dont la composition pondérale après réduction est la suivante :

- cuivre : 44,2 %
- chrome : 1,5 %
- potassium : 1,1 %
- silice : 53,2 %

c'est-à-dire un rapport atomique (Cu/Cr) = 23 et un rapport atomique (K/Cu) = 0,041.

Les conditions opératoires sont très voisines de celles de l'exemple 13 :

température du bain : 204 °C
pression absolue : 8 bars
débit de diméthylamine : 668 g/h (14,81 moles/h)
débit d'éthanol pur : 2 007 g/h (43,57 moles/h)
débit d'hydrogène : 4 883 Nl/h (218 moles/h)

c'est-à-dire avec les rapports molaires suivants :

diméthylamine/éthanol = 0,34
hydrogène/éthanol = 5

Voici les principaux résultats obtenus :

taux de conversion de la diméthylamine : 100 %
rendement en diméthyléthylamine par rapport à la diméthylamine engagée : 91,2 %

Les pourcentages pondéraux de sous-produits rapportés à la diméthyléthylamine fabriquée sont les suivants :

- triméthylamine : 3,8 %
- méthyldiéthylamine : 5,2 %
- méthanol : 0,1 %

Exemple 19

Cet exemple illustre l'influence de la nature du catalyseur sur les résultats. L'essai a été effectué dans un réacteur identique à celui de l'exemple 1, en présence de 2 litres d'un catalyseur dont la composition pondérale avant réduction est la suivante :

- oxyde de cuivre : 42,0 %
- Sesquioxyde de chrome : 42,0 %
- Oxyde de baryum : 9,3 %
- Oxyde de magnésium : 0,5 %
- Oxyde de zinc : 6,2 %

c'est-à-dire avec les rapports atomiques suivants :

- (Cu/Cr) = 0,95
- (Ba/Cu) = 0,116
- (Mg/Cu) = 0,023
- (Zn/Cu) = 0,144

Les conditions opératoires sont les suivantes :

température du bain : 200°C
pression absolue : 8 bars
débit de diméthylamine : 642 g/h (14,25 moles/h)
débit d'éthanol (à 6,4 % d'eau) calculé en éthanol pur : 2 075 g/h (45,04 moles/h)
débit d'hydrogène : 5 040 Nl/h (225 moles/h)

ce qui correspond aux rapports molaires suivants :

diméthylamine/éthanol = 0,32
hydrogène/éthanol = 5

Voici les résultats obtenus :

taux de conversion de la diméthylamine : 100 %
rendement en diméthyléthylamine par rapport à la diméthylamine engagée : 97,5 %

Les pourcentages pondéraux des sous-produits rapportés à la diméthyléthylamine fabriquée sont les suivants :

- triméthylamine : 1,2%
- méthyldiéthylamine : 1,1 %
- triéthylamine : 0,1%
- méthanol : 0,1 %

Exemple 20

Cet exemple illustre l'influence de la nature du catalyseur sur les résultats. L'essai a été effectué dans un réacteur identique à celui de l'exemple 1, en présence de 2 litres d'un catalyseur dont la composition pondérale, après réduction, est la suivante :

- cuivre : 50 %
- oxyde de magnésium : 3 %
- sesquioxyde de chrome : 12 %
- oxyde de zinc : 35 %

c'est-à-dire les rapports atomiques suivants :

- (Cu/Cr) = 5
- (Mg/Cu) = 0,094
- (Zn/Cu) = 0,546

Les conditions opératoires sont très voisines de celles de l'exemple 19 :

température du bain : 200 °C
pression absolue : 8 bars
débit de diméthylamine : 635 g/h (14,09 moles/h)
débit d'éthanol (pur) : 2 072 g/h (44,98 moles/h)
débit d'hydrogène : 5 040 Nl/h (225 moles/h)

ce qui correspond aux rapport molaires suivants:

diméthylamine/éthanol = 0,31
hydrogène/éthanol = 5

Voici les résultats obtenus :

taux de conversion de la diméthylamine : 93,6 %
rendement en diméthyléthylamine par rapport à la diméthylamine transformée : 97,6 %

Les pourcentages pondéraux des sous-produits rapportés à la diméthyléthylamine fabriquée sont les suivants :

- triméthylamine : 0,9 %
- méthyldiéthylamine : 1,0 %
- méthyléthylamine : 0,2 %
- triéthylamine : 0,2 %
- méthanol : 0,2 %

Exemple 21

Cet exemple illustre la possibilité de réduire l'excès d'éthanol par rapport à la diméthylamine engagée. L'essai a été effectué dans un réacteur identique à celui de l'exemple 1, en présence de 2 litres du catalyseur décrit dans l'exemple 20.

Les conditions opératoires sont les suivantes :

température du bain : 200°C
pression absolue : 8 bars
débit de diméthylamine : 344 g/h (7,64 moles/h)
débit d'éthanol pur : 693 g/h (15,03 moles/h)
débit d'hydrogène : 3651 Nl/h (163 moles/h)

ce qui correspond aux rapports molaires suivants :
diméthylamine/éthanol = 0,51
hydrogène/éthanol = 10,8

Voici les résultats obtenus :
taux de conversion de la diméthylamine : 100 %
rendement en diméthyléthylamine par rapport à la diméthylamine : 97,1 %
taux de conversion de l'éthanol théoriquement transformable : 103,9 %
rendement en diméthyléthylamine par rapport à l'éthanol transformé : 97,0 %

Les pourcentages pondéraux des sous-produits rapportés à la diméthyléthylamine fabriquée sont les suivants :

- triméthylamine : 1,5 %
- méthyldiéthylamine : 1,3 %
- triéthylamine : 0,1 %
- méthanol : 0,1 %

## Revendications

1. Procédé de préparation de la diméthyléthylamine par réaction de l'éthanol avec la diméthylamine en présence d'un catalyseur d'hydrogénation-déshydrogénation à base de cuivre, d'oxyde de cuivre et d'oxyde de chrome, contenant éventuellement un ou plusieurs autres oxydes choisis parmi l'oxyde de baryum, l'oxyde de zinc et l'oxyde de magnésium, caractérisé en ce que l'on fait réagir, à une pression comprise entre 1 et 15 bars absolus et à une température comprise entre 180 et 240°C, l'éthanol gazeux avec un mélange gazeux d'hydrogène et de diméthylamine, le rapport molaire diméthylamine/ éthanol étant compris entre 0,20 et 0,60 et le rapport molaire hydrogène/éthanol étant compris entre 3 et 10.

2. Procédé selon la revendication 1 dans lequel la température est comprise entre 200 et 210°C, le rapport molaire diméthylamine/éthanol compris entre 0,25 et 0,40 et le rapport molaire hydrogène/éthanol compris entre 4 et 7.

3. Procédé selon l'une des revendications 1 ou 2, caractérisé par une marche en continu, les gaz séparés de l'effluent réactionnel liquide étant recyclés, 0,1 à 1 % et de préférence 0,2 à 0,7 % en volume de ces gaz de recyclage étant remplacés par de l'hydrogène.

4. Procédé selon l'une des revendications 1 à 3 dans lequel le débit d'éthanol est compris entre 10 et 50 moles/heure par litre de catalyseur, de préférence entre 20 et 25 moles/ heure par litre de catalyseur.

5. Procédé selon l'une des revendications 1 à 4 dans lequel l'effluent du réacteur est traité en phase gazeuse ou liquide sur un catalyseur d'hydrogénation à une température comprise entre 100 et 130°C et à une pression voisine de celle utilisée pour la réaction principale.

6. Procédé selon la revendication 5 dans lequel le catalyseur d'hydrogénation de l'effluent du réacteur est identique à celui utilisé pour la réaction principale.

7. Procédé selon la revendication 5 dans lequel le catalyseur d'hydrogénation de l'effluent liquide du réacteur est du nickel Raney foraminé en morceaux de 2 à 10 mm.

8. Procédé selon la revendication 5 dans lequel le catalyseur d'hydrogénation de l'effluent liquide du réacteur est constitué de nickel déposé sur un support de silice, utilisé en pastilles de 3 à 10 mm.

9. Procédé selon l'une des revendications 1 à 4 où l'effluent du réacteur est traité en continu par de l'hydrate d'hydrazine.

10. Procédé selon l'une des revendications 1 à 9 où la réaction est conduite en présence d'une masse catalytique ayant subi une régénération consistant en une oxydation par un mélange d'air dilué dans de l'azote suivie d'une réduction par un mélange d'hydrogène et de vapeur d'eau.

## Patentansprüche

1. Verfahren zur Herstellung von Dimethyläthylamin durch Reaktion von Äthanol mit Dimethylamin in Gegenwart von Hydrierungs-Dehydrierungs-Katalysatoren auf der Basis von Kupfer, Kupferoxid, Chromoxid und gegenbenenfalls ein oder mehrere andere Oxiden unter den Oxiden des Bariums, Zinks, Magnesiums, dadurch gekennzeichnet, daß das gasförmige Athanol bei Drücken zwischen 1 und 15 bar absolut und Temperaturen zwischen 180 und 240°C mit einem gasförmigen Gemisch von Wasserstoff und Dimethylamin reagiert, wobei das Molverhältnis Dimethylamin/Äthanol zwischen 0,2 und 0,6 und das Molverhältnis Wasserstoff/Äthanol zwischen 3 und 10 liegt.

2. Verfahren nach Anspruch 1, in dem die Temperatur zwischen 200 und 210°C, das Molverhältnis Dimethylamin/Äthanol zwischen 0,25 und 0,40 und das Molverhältnis Wasserstoff/Äthanol zwischen 4 und 7 liegt.

3. Verfahren nach einem der Ansprüche 1 oder 2, gekennzeichnet durch eine kontinuierliche Arbeitsweise, bei der die vom austretenden flüssigen Reaktionsprodukt abgetrennten Gase zurückgewonnen werden und 0,1 bis 1, vorzugsweise 0,2 bis 0,7 Volumenprozent dieser wiederverwendeten Gase durch Wasserstoff ersetzt werden.

4. Verfahren nach einem der Ansprüche 1 bis 3, in dem die Durchflußmenge des Äthanols zwischen 10 und 50 Mol/Stunde je Liter des Katalysators, vorzugsweise zwischen 20 und 25 Mol/Stunde je Liter des Katalysators beträgt.

5. Verfahren nach einem der Ansprüche 1 bis 4, in dem das den Reaktor verlassende Produkt in gasförmiger oder flüssiger Phase auf einem Hydrierungskatalysator bei einer Temperatur zwischen 100 und 130°C und einem Druck, ähnlich dem in der Hauptreaktion verwendeten behandelt wird.

6. Verfahren nach Andoruch 5, in dem der Hydrierungskatalysator für die den Reaktor verlassende FLüssigkeit gleich dem in der Hauptreaktion verwendeten ist.

7. Verfahren nach Ansoruch 5, in dem der Hydrierungskatalysator für die den Reaktor verlassende Flüssigkeit Raney-Nickel in Stücken von 2 bis 10 mm ist.

8. Verfahren nach Anspruch 5, dadurch gekennzeichnet, in dem der Hydrierungskatalysator für die den Reaktor verlassende Flüssigkeit aus Nickel besteht, das auf einem Träger aus Siliziumdioxyd angeordnet ist, der in Tabletten von 3 bis 10 mm verwendet wird.

9. Verfahren nach einem der Ansprüche 1 bis 4, bei dem die den Reaktor verlassende Flüssigkeit kontinuierlich mit Hydrazinhydrat behandelt wird.

10. Verfahren nach einem der Ansprüche 1 bis 9, bei dem die Reaktion in Gegenwart einer Katalysatormasse durchgeführt wird, die einer Regenerierung, bestehend aus einer Oxidation durch eine Mischung von mit Stickstoff verdünnter Luft, gefolgt von einer Reduktion mit einem Gemisch von Wasserstoff und Wasserdampf unterzogen worden ist.

## Claims

1. Process for the preparation of dimethylethylamine by reacting ethanol with dimethylamine, in the presence of hydrogenation/dehydrogenation catalysts based on copper, copperoxide, chromium oxide and, if appropriate, on one or more other oxides amongst barium oxide, zinc oxide, magnesium oxide, characterised in that gaseous ethanol reacts with a gaseous mixture of hydrogen and dimethylamine at absolute pressures between 1 and 15 bars and at temperatures between 180 and 240°C, the molar ratio dimethylamine/ethanol being between 0.2 and 0.6 and the molar ratio hydrogen/ethanol being between 3 and 10.

2. Process according to Claim 1, in which the temperature is between 200 and 210°C, the molar ratio dimethylamine/ethanol is between 0.25 and 0.40 and the molar ratio hydrogen/ethanol is between 4 and 7.

3. Process according to one of Claims 1 or 2, characterised by a continuous operation, the gases separated from the liquid reaction effluent being recycled, and 0.1 to 1 % and preferably 0.2 to 0.7 % by volume of these recycling gases being replaced by hydrogen.

4. Process according to one of Claims 1 to 3, in which the flow rate of ethanol is between 10 and 50 mols/hour per litre of catalyst and preferably between 20 and 25 mols/hour per litre of catalyst.

5. Process according to one of Claims 1 to 4, in which the effluent from the reactor is treated in the gas or liquid phase on a hydrogenation catalyst, at a temperature between 100 and 130°C and at a pressure similar to that used for the main reaction.

6. Process according to Claim 5, in which the hydrogenation catalyst for the effluent from the reactor is identical to that used for the main reaction.

7. Process according to Claim 5, in which the hydrogenation catalyst for the liquid effluent from the reactor is Raney nickel in 2 to 10 mm pieces.

8. Process according to Claim 5, in which the hydrogenation catalyst for the liquid effluent from the reactor consists of nickel deposited on a silica support, used in 3 to 10 mm pellets.

9. Process according to one of Claims 1 to 4, in which the effluent from the reactor is treated continuously with hydrazine hydrate.

10. Process according to one of Claims 1 to 9, in which the reaction is carried out in the presence of a catalyst mixture which has been subjected to a regeneration consisting of oxidation by a mixture of air diluted with nitrogen, followed by reduction by a mixture of hydrogen and steam.

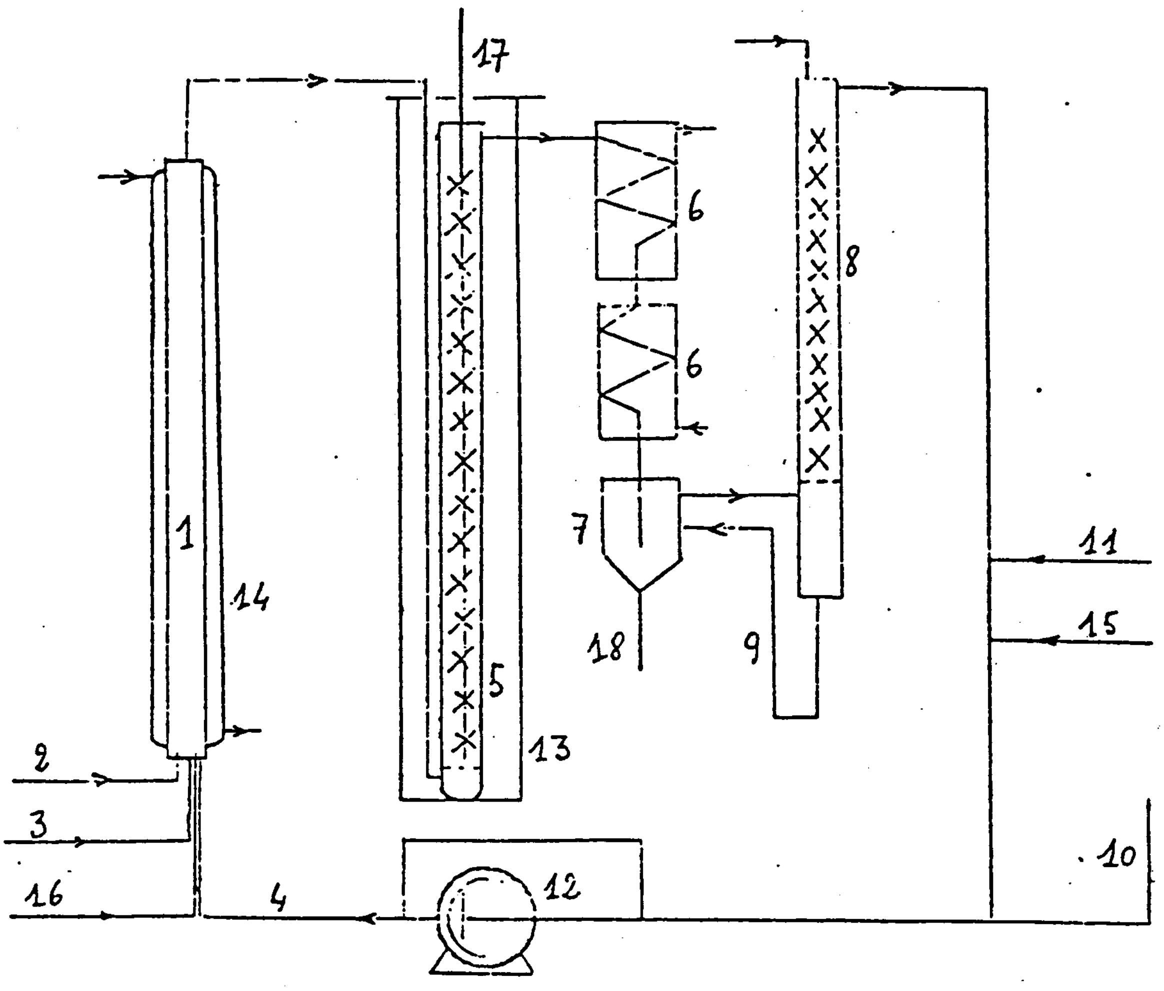
17
6
6
8
1
7
11
14
15
18
9
5
13
2
3
10
16
4
12